(19) **Europäisches Patentamt**
**European Patent Office**
**Office européen des brevets**

(11) **EP 3 645 072 B1**

(12) **EUROPEAN PATENT SPECIFICATION**

(45) Date of publication and mention
of the grant of the patent:
**27.07.2022 Bulletin 2022/30**

(21) Application number: **18734228.2**

(22) Date of filing: **27.06.2018**

(51) International Patent Classification (IPC):
**A61M 1/34** *(2006.01)*    **A61M 1/36** *(2006.01)*
**A61M 5/14** *(2006.01)*    **A61M 39/10** *(2006.01)*

(52) Cooperative Patent Classification (CPC):
**A61M 1/342; A61M 1/3672; A61M 39/10;**
A61M 5/1413; A61M 2206/14

(86) International application number:
**PCT/EP2018/067277**

(87) International publication number:
**WO 2019/002382 (03.01.2019 Gazette 2019/01)**

(54) **ANOTHER INSERT PIECE FOR A BLOOD TUBING SET TO PROMOTE MIXING AN INFUSION SOLUTION WITH A FURTHER FLUID**

EIN WEITERES EINSATZSTÜCK FÜR EINEN BLUTTUBUSSATZ ZUR FÖRDERUNG DER MISCHEN EINES INFUSIONSLÖSUNG MIT EINER WEITEREN FLÜSSIGKEIT

AUTRE ÉLÉMENT D'INSERT POUR UN ENSEMBLE DE TUBULURES POUR LE SANG AFIN DE FAVORISER LE MÉLANGE D'UNE SOLUTION DE PERFUSION AVEC UN AUTRE FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2017 EP 17178458**

(43) Date of publication of application:
**06.05.2020 Bulletin 2020/19**

(73) Proprietor: **Fresenius Medical Care Deutschland GmbH**
**61352 Bad Homburg (DE)**

(72) Inventors:
• **BENELLI, Davide Maria**
**26013 Crema (IT)**

• **FINI, Massimo**
**41037 Mirandola (IT)**
• **REITER, Reinhold**
**26013 Crema (IT)**

(74) Representative: **Bobbert & Partner Patentanwälte PartmbB**
**Postfach 1252**
**85422 Erding (DE)**

(56) References cited:
**WO-A1-2014/026771**    **WO-A1-2018/055091**
**US-A1- 2010 168 643**    **US-A1- 2013 150 772**
**US-B2- 7 931 612**

**Description**

The present invention relates to an insertion piece according to the preamble of claim 1, an extracorporeal blood tubing set according to the preamble of claim 11 and a blood treatment apparatus according to the preamble of claim 15.

During the extracorporeal blood treatment, infusion solutions or medicaments are mostly infused through the extracorporeal blood circuit, i.e., the utilized blood tubing system. Depending on the type of the infusion solution, a fast mixing of the infusion solution with the blood may be favorable or desirable.

For example, anticoagulation infusion solutions are regularly infused in an extracorporeal blood treatment to prevent a possible occlusion of the extracorporeal blood circuit.

Two methods are mainly used for this purpose; the system and the regional anticoagulation. A citrate solution, which complexes calcium, and thus suppresses the blood coagulation, is mostly used as an anticoagulant in the regional anticoagulation. Prior to returning blood to the patient, an additional calcium must be substituted, since too-low calcium concentrations influence nerves and muscles, the blood coagulation and functions of lung, heart and kidneys. In the regional anticoagulation, a calcium-containing solution, by which the physiological calcium concentration in the system blood may be maintained, is therefore added to the blood before reinfusing it into the patient.

Infusing infusion solutions or medicaments into the tubing set takes place usually through addition sites with a T-form, the so-called Tees. Laminar flow conditions predominate in these Tees due to the circular cross section and the smooth inner wall at the addition site. In addition, the flow rates of the infused solutions are low in comparison with the blood flow.

The largely laminar flow conditions and the low flow rates of the infusion solutions or medicaments flowing into the blood stream may delay or slow down the mixing of the blood with the added infusion. This slow mixing of both liquids at the addition site is undesired, especially when adding the calcium solution to blood, and may lead to clot problems at the addition site, due to the punctually continued higher calcium concentrations in blood. To prevent that, a fast and homogenous mixing of the added calcium solution with the blood is desirable.

The same problem may however also occur when mixing other liquids, e.g. medicaments, the fast mixing of which may as well be advantageous.

This problem is known in the prior art. In order to solve it, special addition sites, which have means for generating turbulences, have been described.

For example, an insert piece in form of a T-piece having a spiral structure for a blood tubing set is thus described in WO 2014/026771 A1. The spiral structure serves generating turbulences in the area of the infusion site. The turbulences serve or contribute to a better mixing of the fluids which are brought together.

Furthermore, there are solutions which generate a pulsed infusion flow of the infusion solution into the blood through intermittent operation of an infusion pump.

From US 2010/0168643 A1 an infusion site for improved mixing of fluids is known.

A bypass valve is disclosed in US 7,931,612 B2.

From US 2013/0150772 A1 a by-pass shunt to reduce flow output of a circulatory assist device is known.

An insert piece for a blood line set for promoting the mixture of an infusion solution with another fluid is disclosed in WO 2018/055091 A1.

An object of the present invention may be to propose a further solution for promoting a mixing of an infusion solution with a further fluid, e.g. blood.

The object may be solved by an insert piece having the features of claim 1, an extracorporeal blood tubing set having the features of claim 11 and a blood treatment apparatus having the features of claim 15.

The insert piece according to the present invention is intended to be inserted into a blood tubing set (or into a blood tubing system or an extracorporeal blood circuit) or to be part thereof, respectively. The insert piece comprises at least a first connection site for connecting a first tubing portion of the blood tubing set to the insert piece. It comprises a second connection site for connecting a second tubing portion of the blood tubing set to the insert piece. It comprises a third connection site for connecting a third tubing portion of the blood tubing set to the insert piece. It comprises a first main line for conducting blood as a first liquid through the insert piece. The first main line is in fluid communication with at least the first connection site and with the second connection site or with a lumen surrounded or formed by the first connection site and/or the second connection site.

The insert piece comprises at least a second main line for conducting blood as the first liquid through the insert piece. Like the first main line, the second main line is also in fluid communication with the first connection site and with the second connection site.

The insert piece comprises at least one secondary line for conducting, directly or indirectly, a second liquid, preferably an infusion solution, into an element from the group consisting of the first main line, the second main line and a connection portion which connects both the first main line and the second main line to each other or to the second connection site.

The secondary line is in fluid communication with the third connection site or with a lumen surrounded or delimited by it.

The first connection site comprises a perfusable or flow-through lumen having a first cross section area. The second connection site comprises a perfusable or flow-through lumen having a second cross section area. At least one of the first main line and the second main line comprises a perfusable lumen having a third cross section area. The secondary line comprises a lumen portion. The lumen portion comprises at least one opening or outlet opening for the second fluid.

The blood tubing set, extracorporeal blood circuit or blood tubing system comprises at least one insert piece according to the present invention.

In some embodiments, the insert piece according to the present invention is pressed or interposed between tubing portions of the blood tubing set or it is integrally manufactured therewith.

In some embodiments, the insert piece according to the present invention is firmly connected to at least one of the tubing portions of the blood tubing set, in others, it is releasably connected.

The blood treatment apparatus is connected to at least one blood tubing set according to the present invention.

All embodiments mentioned herein may be exemplary embodiments according to the present disclosure.

Embodiments mentioned herein may encompass one or several of the aforementioned or following features in any arbitrary combination unless the skilled person finds a particular combination as technically impossible. Embodiments are moreover subject-matter of the dependent claims.

Whenever numerical words are mentioned herein, the person skilled in the art shall recognize or understand them as indications of numerical lower limits. Unless it leads the person skilled in the art to an evident contradiction, the person skilled in the art shall comprehend the specification for example of "one" encompassing "at least one". This understanding is also equally encompassed by the present disclosure as the interpretation that a numeric word, for example, "one" may alternatively mean "exactly one", wherever this is evidently technically possible for the person skilled in the art. Both are encompassed by the present disclosure and apply herein to all used numerical words.

Spatial information given herein such as "top", "bottom" etc. refer in case of doubt to the illustration as seen in the accompanying figures and as the position of the device in question when being used by the skilled one.

Whenever it is referred to a cross section or cross section area of an element (such as a line) herein, the cross section or cross section area may be the only one, just one, the average one, the one in the middle section of the element, or the prevailing one.

In certain embodiments, the first connection site, the second connection site and/or the connection portion comprise, respectively, a mostly cylindrical inner wall or a section having a cylindrical inner wall.

In certain embodiments, the insert piece is manufactured integrally with the blood tubing set. All or some of the sections of the insert piece denoted herein as connection sites are in such embodiments passage sections between insert piece and adjacent or continuing tubing portions.

In certain embodiments, the secondary line is connected in fluid communication with, or comprises, a source for the infusion solution.

In certain embodiments, the infusion solution is a calcium solution or comprises a calcium solution. The present disclosure is however not limited to using a calcium-containing solution. Other medicament solutions are encompassed by the present disclosure as well. They include, in particular, citrate solution.

In certain embodiments, the third cross section area is, as a maximum, half, or substantially half, as large, as the first cross section area and/or the second cross section area. "Substantially half as large" may refer to or take on a value between 35% and 65%. All intermediate values and in particular each integer percentage value (36%, 37%, 38%, etc.) are included or contemplated as well.

In certain embodiments, the cross section area of the first main line and the cross section area of the second main line taken together or their sum are smaller than the cross section of the first connection site, the second connection site and/or the cross section of tube section of the blood tube set connected to the insert piece. With this design, the flow of the first liquid can be speed up within the first and/or the second main line which in turn contributes to generating turbulences and to prevent poor mixing.

In some embodiments, the cross section area of the first main line and the cross section area of the second main line taken together or their sum equal the cross section of the first connection site, the second connection site and/or the cross section of tube section of the blood tube set connected to the insert piece. With this design, turbulences may be avoided upon entry of the fluid into the first main line and the second main line. However, turbulences are first generated further downstream upon entry of the second fluid into the first fluid at the mouth or opening of the secondary line. The turbulences achieved as intended there may be used to prevent poor mixing.

The entrance into the first main line and/or into the second main line can optionally be tapered or result in another way in a decreased or decreasing cross section. A such amended cross section (or cross section area, which terms may be interchangeable) may advantageously cause disturbance in the flow of the first liquid and turbulences. Turbulence, in turn, may contribute to an enhanced mixing of the first and the second liquids further downstream. The same applies optionally to the exit from the first main line and/or from the second main line.

Also, the cross sections need not to be increased or increasing or decreased or decreasing by deviations from, e. g., a circular cross section or need not be homogeneously altered along their entire circumference. Rather, amending parts of the circumference only may already lead to the desired turbulences.

In certain embodiments, a baffle or deflector element is provided in the area of the connection portion and in the interior of the latter. This element is arranged such that a second liquid flowing out of the outlet opening or opening of the secondary line is limited in its radial movement or in its movement in the outlet direction.

In certain embodiments, the baffle or deflector element is not the inner wall of the main line, for example in the area of the first connection site, of the second connection site or the connection portion.

In certain embodiments, an addition line is connected in fluid communication to a blood return line and/or to a blood withdrawal line of the blood tubing set via the secondary line of or connected to the insert piece.

In certain embodiments, the blood tubing set is suitable and/or provided for executing a regional anticoagulation.

In certain embodiments, the blood tubing set is suitable and/or provided for executing a hemodialysis, a hemofiltration or a hemodiafiltration or a plasmapheresis treatment or a whole blood adsorption treatment.

In some embodiments, the connection portion is arranged - in flow direction of the first liquid - between the first main line and/or the second main line on the one hand and the second and/or the third connection site on the other. Hence, it may be arranged downstream to the first and/or the second main line. It may be arranged upstream to the second and/or the third connection site.

In some embodiments, the connection portion comprises, in flow direction towards the second connection site or up-stream to the second connection site, a cross section, in particular a conical one, which widens, increases or enlarges along the flow direction.

In some embodiments, there is a through-opening of the insert piece arranged between or delimited or framed by sections of the first main line and sections of the second main line.

In some embodiments, the through-opening has a round, oval or oblong hole form or shape. Alternatively, it comprises a section which has a round, oval or oblong hole form.

In some embodiments, the insert piece consists of two or at least two components, which are welded and/or glued with or to each other. For example, the tubes may be glued or welded to the insert piece. Also, the insert piece may consist of, e. g., an upper part and a lower part which are optionally glued one to the other. Alternatively, or in addition, they may be interconnected to each other by other methods such as clamping and the like.

In some embodiments, the insert piece is integrally formed, e. g. by injection molding. The tubes may optionally be clamped and/or glued to the insert piece. This may be the simplest ways for assembling the components to each other. Also, a welding process may be waived this way.

In some embodiments, at least one of the first main line and/or the second main line, or sections thereof, respectively, are tubes or channels.

The first main line and/or the second main line may, in particular, be those which are inserted into or integral with tube intakes or connectors which are in fluid communication with the first connection site or with the second connection site, and which are optionally integrally produced therewith.

In some embodiments, at least one of the first main line and the second main line are integral parts of the insert piece or integrally with the latter.

In certain embodiments, the insert piece is made of plastic, preferably injection molded.

In certain embodiments, the insert piece has no three-dimensional spiral structure for generating turbulences, in particular no recessed section in the inner wall of the connection portion.

In some embodiments, the first, the second and/or the third connection site is bonded or connected to a tubing portion, respectively.

In some embodiments, the blood tubing set may comprise at least one of a drip chamber, further insert pieces in form of Tees or injection points, and the like.

When liquids are mentioned herein, it is not to be understood restrictively. The present invention encompasses or contemplates also bringing together other fluids in a wider sense.

In some embodiments, the first main line and the second main line have two, in particular only two, common junction portions or connection positions, e. g. the first and the second connection sites. In certain embodiments, the lumen of the first main line and the lumen of the second main line are physically separated from each other. Physically separated from each other may mean that there are walls or other elements that separate the flow through the first main line from the flow through the second main line such that the fluid flowing through the first main line may not mix with the fluid flowing through the second main line. In some embodiments, this applies at least along the larger part of both the first main line and the second main line, i.e., the first main line and the second main line are separated though their lumens are in fluid combination with each other at both a first end section and a second end section of both first main line and the second main line, respectively. The end sections may be the entrance into and the exit from the first main line and the second main line, respectively.

According to the present invention, the first main line and the second main line are arranged such that fluid may flow either through the first main line or through the second main line, e. g. since the fluid paths of the first and the second main lines are arranged in parallel one to the other, or next or besides to each other, not however in subsequence or one following the other.

In certain embodiments, neither the first main line nor the second main line comprises a pressure-responsive valve.

In some embodiments, the insert piece does not comprise a flow regulator for controlling the flow through the insert piece or through the first or the second main line, in particular no flow regulator adjustable by the

In certain embodiments, the secondary line is in fluid communication with the lumen of the first main line, the second main line and/or the connection portion. Hence, the first liquid and the second liquid may mix in use.

In some embodiments, the first main line and the second main line define a common plane perpendicular to the main extension direction of the secondary line.

In certain embodiments, both the first connection site and the second connection site extend along a common straight line.

The common straight line may coincide with the direction of the fluid when flowing into and/or out from the insert piece.

In certain embodiments, the cross section of any line, e. g. the first main line or the second main line, refers to the inner cross section or the perfusable cross section of a nose or another flexible tubing that is inserted or will be inserted into the corresponding first, second or third connection site.

In some embodiments, the cross section of the first tubing portion and/or of the first connection site is smaller than the cross section of the second tubing portion or of the second connection site.

In certain embodiments, the first connection site is where the first fluid enters the insert piece during use.

In some embodiments, the second connection site is closer to the secondary line than the first connection site.

In certain embodiments, the cross section of the first main line and/or of the second main line is bigger than, or equals, 50% of the cross section of the first tubing portion or of the first connection site.

In certain embodiments, the sum of the cross sections of the first main line and of the second main line is bigger than the cross section of the first tubing portion or of the first connection site.

In some embodiments, the cross section of the first main line and the cross section of the second main taken together equal the cross section of the first tubing portion or of the first connection site.

In certain embodiments, the first and the second main are arranged such that they form a circle within a plane, the plane preferably extending through the first and the second main.

In certain embodiments, blood entering the insert piece through the first connection site is split up in two flows (by means of the first and the second main lines). The flows are separate from each other by the design of the first and second main lines and rejoin again, preferably near the outlet of the secondary line. That way, turbulences are created.

In some embodiments, the first and the second main lines are advantageously arranged such that they the two flows collide or meet in a more or less frontal direction comparable to a frontal impact between the two flows.

In certain embodiments, the secondary line and/or its third connection site may be perpendicular to a plane spun by the first and the second main lines. However, the opening plane (or mouth) of the secondary line, through which the second fluid exits in order to mix with the first fluid may also be perpendicular to the plane spun by the first and the second main lines; alternatively, the direction along which the second fluid exits from the secondary line in order to mix with the first fluid may be parallel to or part of the plane spun by the first and the second main lines.

In certain embodiments, inside of the mouth piece of the secondary line the second fluid provided by the secondary line has to change its travelling direction in order to exit from the secondary line and to inter into the first fluid.

In certain embodiments, the secondary line and/or its third connection site is perpendicular to the plane spun by the first and the second main lines. However, the opening plane (or mouth) of the secondary line, through which the second fluid exits in order to mix with the first fluid may also be parallel to the plane spun by the first and the second main lines; alternatively, the direction along which the second fluid exits from the secondary line in order to mix with the first fluid may be perpendicular to the plane spun by the first and the second main lines.

In certain embodiments, inside of the mouth piece of the secondary line the second fluid provided by the secondary line has not to change its travelling direction in order to exit from the secondary line and to inter into the first fluid.

The effect of the design according to the present invention was tested by the inventors under the following simulation conditions:

As "blood solution", 1,5 l $H_2O$ and 0,5 l Glycerol having a density of 1063.4 kg/m$^3$ and a viscosity of 0.3536 Pas was used. As "Calcium solution" $H_2O$ having a density of 997.561 kg/m$^3$ and a viscosity of 8.8871 e-4 Pas was used.

The software used for simulation was "Hypermesh-ANSA" and "Star CCM+".

The speed range was 50ml/min for the "blood" substitute and 10ml/hour for the "Calcium" solution substitute.

At different sections (orthogonal planes at the axis of the outlet tube with differing distance to the outlet opening or mouth), the flow distribution of the tracer was calculated with the formula below. The section of the tube is considered as a mesh, formed by i-th cells:

$$\gamma = 1 - \frac{\Sigma Ai \cdot \sqrt{(Vi - Vmin)^2}}{2 \cdot Vmin \cdot A}$$

Wherein:

Y:       Tracer distribution
Ai:      Area of the single i-th cell of the section
A:       Total area of the section
Vi:      Volumetric fraction of the solution of calcium, calculated in the single generic cell i
Vmin:    Minimum value of the volumetric fraction on the Ca solution in the section

If Vi is similar to Vmin (therefore Vi-Vmin tends to 0) then the volumetric fraction of calcium is similar in all i cells of the section, which stands for a good mixing result.

If $\gamma$ tends to 1 a complete mixing of the tracer (calcium) is assumed.

If Y tends to 0 no mixing of tracer (calcium) has taken place.

The surface uniformity/tracer distribution computed by means of above formula was: 0.71 at a site 0.5 cm downstream from where the "Calcium solution" was introduced into the "blood", 0.84 at a site 1 cm downstream, 0.88 at a site 2 cm downstream, 0.91 at a site 3 cm downstream, 0.93 at a site 4 cm downstream, 0.95 at a site 5 cm downstream, 0.96 at a site 6 cm downstream from where the "Calcium solution" was introduced into the "blood".

Some or all of the embodiments according to the present invention may include one or several of the aforementioned or following advantages.

One advantage is that blood clots resulting from improper mixing of the first and the second fluids may be prevented. Hence, the treatment needs not to be interrupted because of clotting and/or occlusions.

According to another advantage no further devices or method steps are required for ensuring a swirling of the infusion solution within the blood due to the particular shape or design of the insert piece described herein. Nevertheless, an accelerated and reliable mixing of the infusion solution with the blood may be achieved.

An advantage of using the insert piece according to the present invention may further be that the herein described advantages may be achieved without requiring a change in the controlling of the blood treatment apparatus or the pump for the infusion solution. Rather, the structure of the insert piece effects an advantageous distribution of the second fluid into the first fluid without requiring, e. g., a pulsating addition as described in, e. g., DE 10 2013 011 010 A1. The effect according to the present invention may be achieved by the geometry of the insert piece described herein alone. In particular, no amendments to the controlling of the infusion pump are required. Its conveying characteristics may be maintained.

This advantageously implies that no structural or other type of adjustment of the already purchased blood treatment apparatus, e. g. a software adjustment or modification, is required. The implementation of the present invention may consist of using the insert pieces or blood tubing sets as set forth herein. Switching to such blood tubing sets is also easy to accomplish since they are anyhow single-use articles or disposables.

The effect of the insert piece according to the present invention is independent of parameters of the blood treatment. If for example the conveying rate of the blood pump and the conveying rate of the pump for the infusion solution being in line with the former are changed, then the effect principle or the effect of the insert piece according to the present invention remains unchanged or undisturbed.

Also, as stated above, the design of the insert piece according to the present invention allows for a thorough mixing of the second fluid introduced into the first fluid. That way, the second fluid may be used in a higher concentration as in the prior art; due to the better mixing of the fluids no or less adverse effects may be observed even when the second fluid, such as a calcium solution, is used in a relatively high concentration. Also, due to the good mixing features of the insert piece according to the present invention the second fluid may be introduced at comparably low speed.

Finally, the insert piece convinces or impresses due to its extremely simple and, when desired, even symmetrical design. It can be produced by most simple injection molding tools which may reduce the total costs of its production significantly.

The present invention is exemplarily explained in the following, regarding the accompanying drawings in which identical reference numerals denote the same or similar elements. The following applies in the partially highly simplified figures:

Fig. 1     shows the insert piece in a first exemplary embodiment;

Fig. 2     shows the insert piece of Fig. 1 in a first cut in the longitudinal direction;

Fig. 3     shows the insert piece of Fig. 1 in a second cut in the longitudinal direction;

Fig. 4     shows the insert piece in a second exemplary embodiment;

Fig. 5     shows the insert piece similar to that of Fig. 4 in a first cut in the longitudinal direction in a plan view;

Fig. 6     shows the lower part of the insert piece of Fig. 4 in a second cut in the longitudinal direction in perspective from above;

Fig. 7     shows the upper part of the insert piece of Fig. 4 in a second cut in the longitudinal in perspective direction from below;

Fig. 8     shows the insert piece according to a third exemplary embodiment in a first cut in the longitudinal direction;

Fig. 9      shows a partly cut insert piece according to a fourth exemplary embodiment;

Fig. 10     shows the insert piece of Fig. 9 in a longitudinal section;

Fig. 11     shows a blood tubing set according to the present invention having an insert piece according to the present invention;

Fig. 12     shows an insert piece according to a fifth exemplary embodiment in a longitudinal section;

Fig. 13a    shows the insert piece of Fig. 12 from behind; and

Fig. 13b    shows the insert piece of Fig. 12 from its front side.

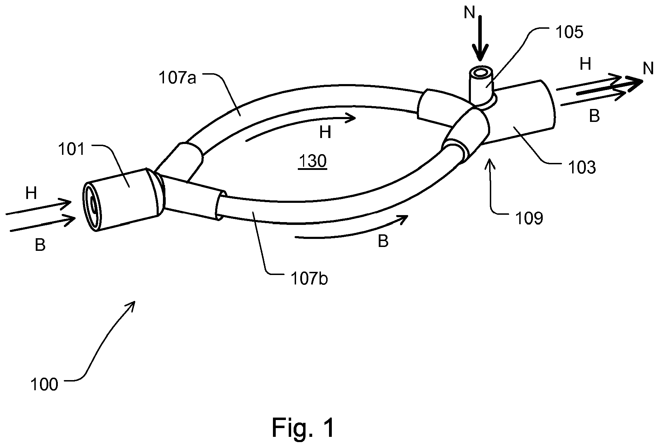

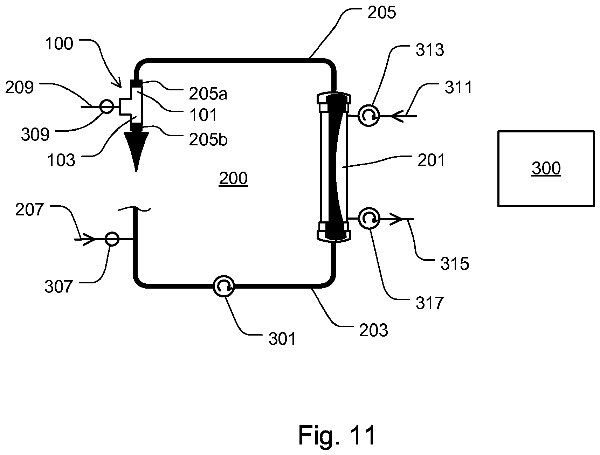

**Fig. 1** shows an insert piece 100 in a first exemplary embodiment according to the present invention for a blood tubing set 200, not shown in Fig.1 (see however Fig. 11).

The insert piece 100 comprises a first connection site 101 by means of which a first tubing portion 205a (see Fig. 11) of the blood tubing set 200 may be connected to the insert piece 100.

The insert piece 100 comprises a second connection site 103 by which a second tubing portion 205b (see Fig. 11) of the blood tubing set 200 may be connected to the insert piece 100.

The insert piece 100 comprises a third connection site 105 by which a third tubing portion, here it is a line 209 for calcium solution of the blood tubing set 200 (see Fig. 11), may be connected to the insert piece 100.

The insert piece 100 comprises a first main line 107a for conducting a first liquid, preferably blood, through the insert piece 100. The first main line 107a is in fluid communication with the first connection site 101 and with the second connection site 103. The first liquid may flow in direction of the arrows H into and through the first main line 107a.

The insert piece 100 comprises also at least a second main line 107b (and possible even a third, fourth, and so on line) for conducting the first liquid through the insert piece 100. The second main line 107b is in fluid communication with the first connection site 101 and with the second connection site 103. The first liquid may flow in direction of the arrows B into and through main line 107b.

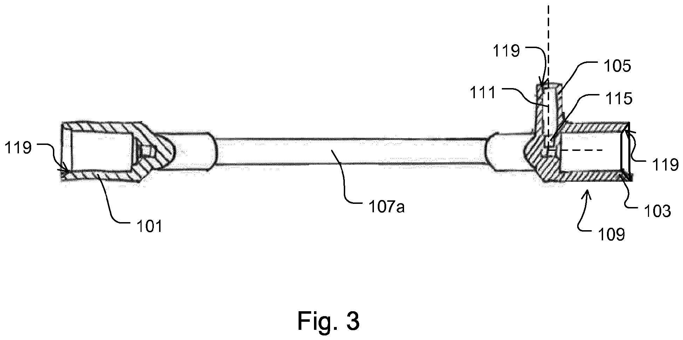

The insert piece 100 comprises an optional junction portion (also referred to as a connection portion herein) 109, in which the first main line 107a, the second main line 107b and a secondary line 111 (see Fig. 3) meet or end. The junction portion 109 may comprise also the second connection site 103 and/or the third connection site 105.

The insert piece 100 comprises a secondary line 111. It serves conducting a second liquid, preferably an infusion solution. The second liquid may flow in direction of the arrow N into the secondary line 111.

The secondary line 111 is in fluid communication with the third connection site 105, and it can, as in Fig. 1, be part of or end with the third connection site 105. It is further in fluid communication with the first and the second main lines 107a, 107b.

The first connection site 101 comprises a perfusable lumen having a first cross section area.

The second connection site 103 comprises a perfusable lumen having a second cross section area.

The first and/or the second main lines 107a, 107b may comprise a perfusable lumen having a third cross section area.

The secondary line 111 comprises a lumen portion which protrudes or opens into the interior of the junction portion 109 and which comprises at least one opening or outlet opening 115. The second liquid may be introduced into a lumen of the junction portion 109 or of another line through the outlet opening 115.

As Fig. 1 further shows, the lumen portion opens into the interior of the junction portion 109.

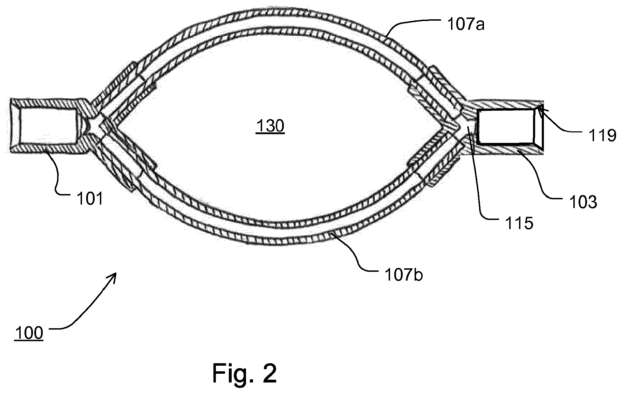

The first connection site 101, the second connection site 103 and/or the third connection site 105 may optionally comprise, respectively, a chamfer 119 of the inner wall or of the outer wall, see Fig. 2 and Fig. 3.

The first and the second main lines 107a, 107b may, as in Fig. 1, delimit an optional through-opening 130 of the insert piece 100.

Hence, blood entering the insert piece 100 through the first connection site 101 is split up in two flows (by means of the first and the second main lines 107a, 107b) that are separate from each other and that rejoin near the outlet of the secondary line 111. That way, turbulences are created.

As can be seen from Fig. 1, in any embodiment according to the present invention - such as the one shown in Fig. 1 without being limited to this particular embodiment - the first and the second main lines 107a, 107b may advantageously arranged such that they the two flows collide or meet in a more or less frontal direction.

**Fig. 2** shows the insert piece 100 of Fig. 1 in a first cut in the longitudinal direction in a plan view.

**Fig. 3** shows the insert piece 100 of Fig. 1 in a second cut in the longitudinal direction.

As can be seen from Fig. 3, in any embodiment according to the present invention - such as the one shown in Fig. 3 without being limited to this particular embodiment - the secondary line 111 and/or its third connection site 105 may be perpendicular to the plane spun by the first and the second main lines 107a, 107b, the plane being in a horizontal or left-right direction in Fig. 3. However, the opening plane (or mouth or outlet opening) of the secondary line 111, through which the second fluid exits in order to mix with the first fluid may also be perpendicular to the plane spun by the first and the second main lines 107a, 107b; alternatively, the direction along which the second fluid exits from the secondary line 111 in order to mix with the first fluid may be parallel to or part of the plane spun by the first and the second main lines 107a, 107b.

Hence, inside of the mouth piece of the secondary line 111 the second fluid provided by the secondary line has to change its travelling direction (indicated in Fig. 3 in a broken line) in order to exit from the secondary line 111 and to inter into the first fluid.

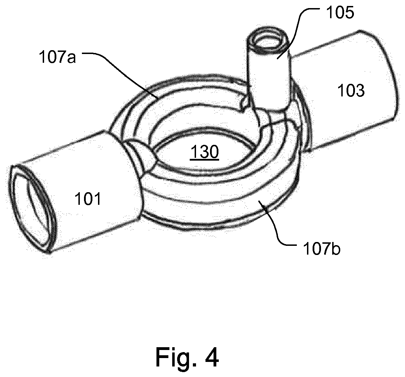

.**Fig. 4** shows the insert piece 100 in a second exemplary embodiment.

In contrast to the embodiment shown in Figs. 1 to 3, the first main line 107a and the second main line 107b are integrally formed parts of the insert piece 100 and, hence, optionally made from the same material as, e.g., the first connection site 101 which facilitates the production process of the insert piece 100. The insert piece 100 can be formed, e. g., by injection molding.

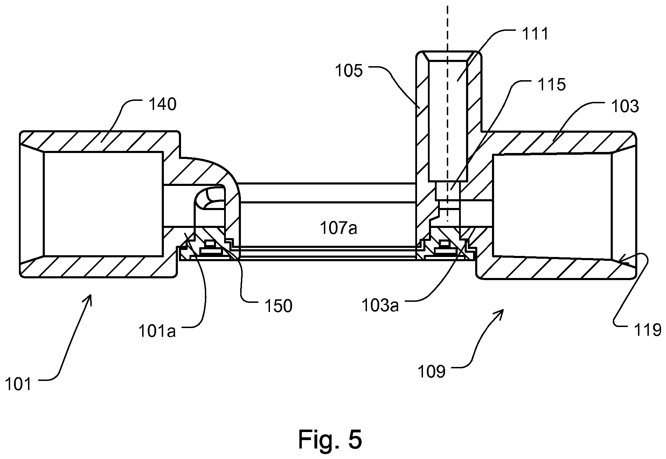

**Fig. 5** shows the insert piece 100 similar to that of Fig. 4 in a first cut in the longitudinal direction.

Reference numeral 101a denotes an entrance into the first main line 107a and/or into the second main line 107b. The entrance 101a can be either part of the first connection site 101 or of the first and/or second main lines 107a, 107b.

Reference numeral 103a denotes an exit from the first main line 107a and/or from the second main line 107b. The exit 103a can be either part of the second connection site 103 or of the first and/or second main lines 107a, 107b.

The presence of an entrance 101a and/or an exit 103a and their designs as discussed above, are, of course, not limited to the second embodiment as shown in Fig. 5. Only, they can be best observed in Fig. 5.

As is exemplarily shown in Fig. 5, the insert piece 100 may comprise or consist of an upper part 140 (shaded in a first manner) and a lower part 150 (shaded differently than the upper part 140) which may be glued one to the other or connected to each other in any other way (such as welding).

As can be seen from Fig. 5, in any embodiment according to the present invention - such as the one shown in Fig. 5 without being limited to this particular embodiment - the secondary line 111 and/or its third connection site 105 may be perpendicular to the plane spun by the first and the second main lines 107a, 107b, the plane being in a horizontal or left-right direction in Fig. 5. However, the opening plane (or mouth) of the secondary line 111, through which the second fluid exits in order to mix with the first fluid may also be parallel to the plane spun by the first and the second main lines 107a, 107b; alternatively, the direction along which the second fluid exits from the secondary line 111 in order to mix with the first fluid may be perpendicular to the plane spun by the first and the second main lines 107a, 107b.

Hence, inside of the mouth piece of the secondary line 111 the second fluid provided by the secondary line has not to change its travelling direction (indicated in Fig. 5 in a broken line) in order to exit from the secondary line 111 and to inter into the first fluid.

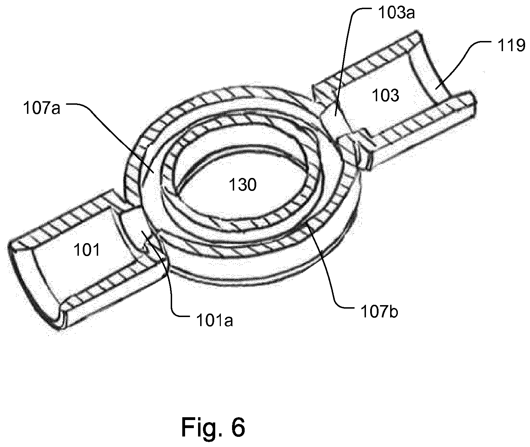

**Fig. 6** shows one part of the insert piece 100 of Fig. 4 in a second cut in the longitudinal direction from above and in slight perspective.

Also, as can be seen from Fig. 6, in any embodiment according to the present invention - such as the one shown in Fig. 6 without being limited to this particular embodiment - the first and the second main lines 107a, 107b may advantageously arranged such that they form a circle within a plane extending through the first and the second main lines 107a, 107b.

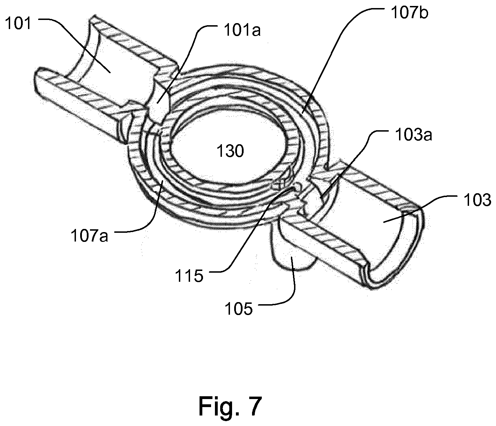

**Fig. 7** shows the other part of the insert piece 100 of Fig. 4 in the second cut in the longitudinal direction from below and in slight perspective.

In the particular embodiment shown in Fig. 7 or in any other embodiment, the cross section area of the first main line 107a and/or the cross section of the second main line 107b is less than half of the cross section area of the first connection site 101 and/or of the second connection site 103.

For example, the first connection site 101 may have a cross section area of 14.51 $mm^2$ (at a diameter of 4.3 mm) whereas the cross section area of the first main line 107a and/or the cross section of the second main line 107b may be 2.83 $mm^2$ (at a diameter of 1.9 mm). Other values for the cross section area of the first main line 107a and/or the cross section of the second main line 107b such as 3.14 $mm^2$ (at a diameter of 2.0 mm) are encompassed as well, of course.

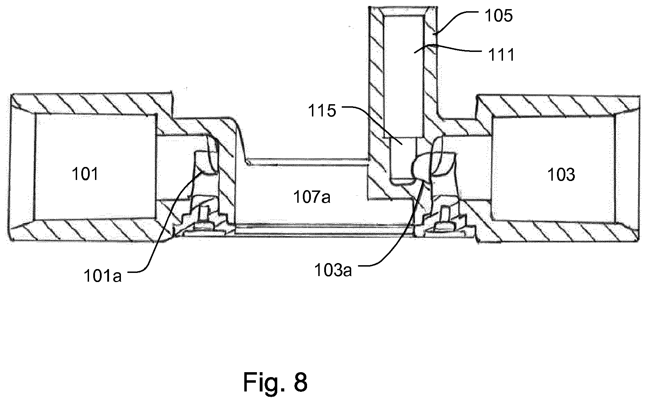

**Fig. 8** shows the insert piece 100 in a third exemplary embodiment in a first cut in the longitudinal direction.

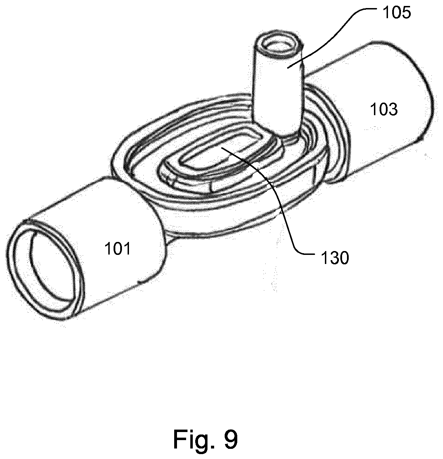

**Fig. 9** shows a partly cut insert piece 100 in a fourth exemplary embodiment.

In contrast to the insert piece 100 of, e. g., Figs. 1 to 3, the through-opening 130 is oval in shape.

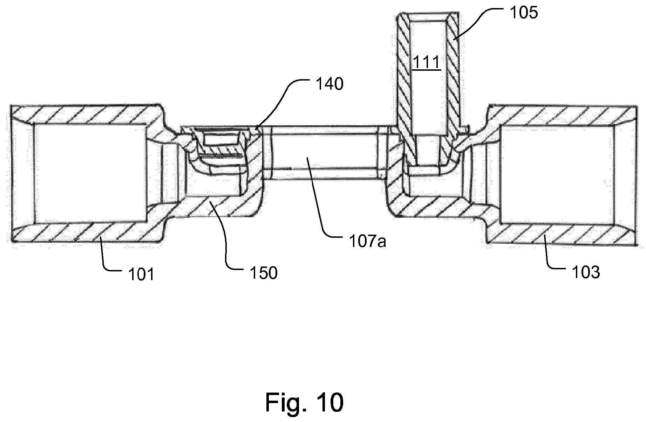

**Fig. 10** shows the insert piece 100 of Fig. 9 in a longitudinal section.

**Fig. 11** shows an exemplary embodiment of an optional basic arrangement of an insert piece 100 as described herein in an extracorporeal blood tubing set 200 according to the present invention, the latter being illustrated in a schematically highly simplified manner.

The blood tubing set 200 comprises a hemofilter 201 or is connected thereto. A blood withdrawal line 203 (or arterial line) and a blood return line 205 (or venous line) are connected to the hemofilter 201 or dialyzer or blood filter.

The blood withdrawal line 203 is operatively connected to, or comprises, a blood pump 301.

A further addition line, here a line 207 for citrate solution, opens upstream of the blood pump 301 into the blood withdrawal line 203.

The line 207 is operatively connected to, or comprises, a citrate pump 307.

Downstream of the hemofilter 201, the line 209 for calcium solution according to the present invention opens into the blood return line 205.

The insert piece 100 according to the present invention is operatively connected to a calcium pump 309 or comprises the latter. It is supplied by a source for infusion solution which is not shown in Fig. 11, herein exemplarily a calcium source. The source may be a bag or a bottle. The infusion solution may optionally be generated on-line; in such case the respective device, in which the infusion solution is generated, is considered as a source.

The operative connection to the calcium pump 309 is to be understood herein as an example. Arranging the insert piece 100 behind another pump than a calcium pump, i.e. for example downstream of a citrate pump like the citrate pump 307 of Fig. 11, is encompassed by the present invention.

The hemofilter 201 is connected to a line 311 for fresh dialysis liquid and to a line 315 for spent dialysate or filtrate. The line 311 is connected to, or comprises, a dialysis liquid pump 313. The line 315 is connected to, or comprises, a filtrate pump 317.

The arrowhead indicates the flow direction when using the blood tubing set 200 as intended.

The blood tubing set 200 shown in Fig. 11 may correspond to a standard extracorporeal blood tubing set and may in particular be suitable for the CVVHD (continuous venovenous hemodialysis).

The pumps 301, 307, 309, 313 and 317 may be part of a blood treatment apparatus 300 which is only schematically indicated. The same applies for the lines 311 and 315.

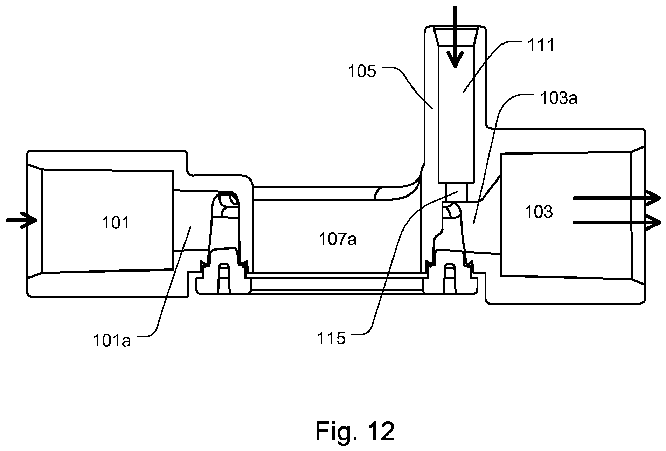

**Fig. 12** shows an insert piece 100 according to a fifth exemplary embodiment in a longitudinal section. The arrows indicate the flow directions into or out of the insert piece 100, either of the blood or the calcium solution or a mixture thereof during use as intended.

In some embodiments, and in particular in the embodiment shown in Fig. 12, the diameter or cross section area of the first connection site 101 (that can be suitable for a 3.5x5.5 mm tube) is less than, or equal to, the diameter or cross section area of the second connection site 103 (that can be suitable for a 4.3x6.8 mm tube). An enlarged lumen closely downstream the connection of the secondary line 111 may possibly decelerate the speed of the flow and, therefore, advantageously allow a more thorough mixing.

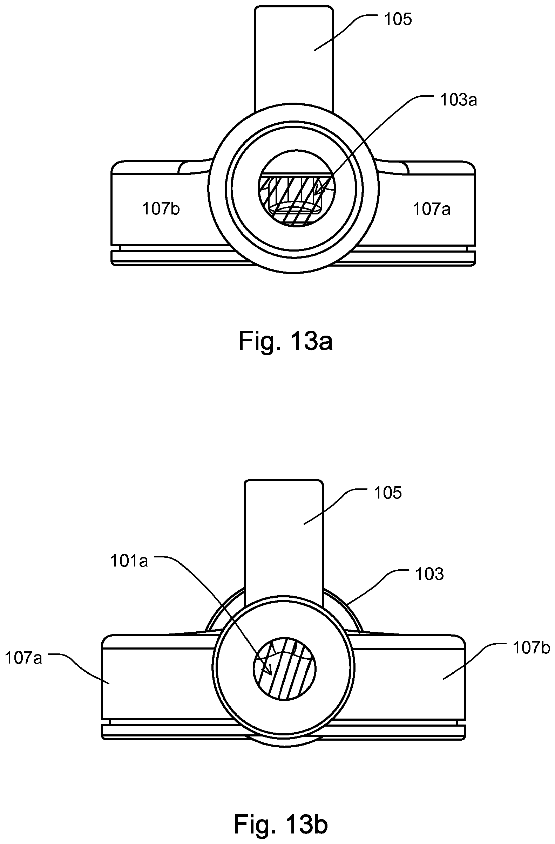

**Fig. 13a** shows the insert piece 100 of Fig. 12 with view to the exit 103a.

It can be seen that the circular exit 103a has a larger diameter than the circular entrance 101a shown in Fig. 13b.

**Fig. 13b** shows the insert piece 100 of Fig. 12 with view to the entrance 101a.

List of reference numerals

| | |
|---|---|
| 100 | insert piece |
| 101 | first connection site |
| 101a | entrance |
| 103 | second connection site |
| 103a | exit |
| 105 | third connection site |
| 107a | first main line |
| 107b | second main line |
| 109 | junction portion or connection portion |
| 111 | secondary line |
| 115 | outlet opening or outlet |
| 119 | chamfer |
| 130 | through-opening |
| 140 | upper part |
| 150 | lower part |
| 200 | blood tubing set, blood tubing system |
| 201 | hemofilter or blood filter or dialyzer |
| 203 | blood withdrawal line |
| 205 | blood return line |
| 205a | first tubing portion |
| 205b | second tubing portion |
| 207 | line for citrate solution |
| 209 | line for calcium solution; addition line; third tubing portion |
| 300 | blood treatment apparatus |
| 301 | blood pump |
| 307 | citrate pump |
| 309 | calcium pump |
| 311 | line for fresh dialysis liquid |
| 313 | pump for fresh dialysis liquid |
| 315 | line for spent dialysate, or filtrate |
| 317 | pump for spent dialysate, or filtrate |
| H | flow direction of the first fluid into and through the first main line |
| B | flow direction of the first fluid into and through the second main line |
| N | inflow direction of the second fluid into the secondary line |

**Claims**

1. An insert piece (100) for a blood tubing set (200), comprising at least, respectively,

   - a first connection site (101) for connecting a first tubing portion (205a) of the blood tubing set (200) to the insert piece (100);
   - a second connection site (103) for connecting a second tubing portion (205b) of the blood tubing set (200) to the insert piece (100);
- a third connection site (105) for connecting a third tubing portion (209) of the blood tubing set (200) to the insert piece (100);
- a first main line (107a) for conducting blood as a first liquid through the insert piece (100), wherein the first main line (107a) is in fluid communication with the first connection site (101) and with the second connection site (103);
- a second main line (107b) for conducting blood as the first liquid through the insert piece (100), wherein the second main line (107b) is in fluid communication with the first connection site (101) and with the second connection site (103);
- a connection portion (109) which connects both main lines (107a, 107b) to each other or to the second connection site (103);
- a secondary line (111) for conducting a second liquid, preferably an infusion, into at least one of

the first main line (107a);
the second main line (107b); and
the connection portion (109);
wherein

- the secondary line (111) is in fluid communication with the third connection site (105);
- the first connection site (101) comprises a perfusable lumen having a first cross section area;
- the second connection site (103) comprises a perfusable lumen having a second cross section area;
- the first main line (107a) and/or the second main line (107b) comprises a perfusable lumen having a third cross section area; and
- the first main line (107a) and the second main line (107b) being arranged such that fluid is conducted through the insert piece (100) either through the first main line (107a) or the second main line (107b).

2. The insert piece (100) according to claim 1, wherein the sum of a cross section area of the first main line (107a) and a cross section area of the second main line (107b) is smaller than the cross section area of the first connection site (101), the second connection site (103) and/or the cross section area of tube section of the blood tubing set (200) connected to the insert piece (100).

3. The insert piece (100) according to claim 1, wherein the secondary line (111) is connected in fluid communication to, or comprises, a source for the infusion solution.

4. The insert piece (100) according to any one of the preceding claims, wherein the third cross section area is maximally half as large or substantially half as large as the first cross section area and/or the second cross section area.

5. The insert piece (100) according to any one of the preceding claims, wherein the connection portion (109) is arranged in flow direction of the first liquid between the first main line (107a) and/or the second main line (107b) on the one side, and the second connection site (103) and/or third connection site (105) on the other.

6. The insert piece (100) according to any one of the preceding claims, wherein the connection portion (109) comprises, in flow direction towards the second connection site (103), a cross section, in particular a conical one, which widens or enlarges along the flow direction.

7. The insert piece (100) according to any one of the preceding claims, wherein a through-opening (130) of the insert piece (100) is arranged between sections of the first main line (107a) and sections of the second main line (107b).

8. The insert piece (100) according to any one of the preceding claims, consisting of two or at least two components, which are welded and/or glued with or to each other.

9. The insert piece (100) according to any one of the preceding claims, wherein the first main line (107a) and/or the second main line (107b), or sections thereof, respectively, are tubes, in particular those inserted into tube intakes which are in fluid communication with the first connection site (101) or with the second connection site (103) and are integrally produced therewith.

10. The insert piece (100) according to any one of the preceding claims, wherein the first main line (107a) and/or the second main line (107b) are integral parts of the insert piece (100).

11. A blood tubing set (200) comprising at least one insert piece (100) according to any one of the preceding claims, wherein the insert piece (100) is connected in fluid communication with an addition line (207, 209) and with a blood withdrawal line (203) and/or with a blood return line (205).

12. The blood tubing set (200) according to claim 11, which is suitable and/or prepared for executing a regional anticoagulation.

13. The blood tubing set (200) according to any one of claims 11 to 12, wherein the blood tubing set (200) is suitable for executing a hemodialysis, a hemofiltration, a hemodiafiltration, a plasmapheresis treatment or a whole blood adsorption treatment.

14. The blood tubing set (200) according to any one of claims 11 to 13, wherein the first connection site (101) and the second connection site (103) are connected to a blood-return line (205) of the blood tubing set (200), and wherein the third connection site (105) is connected to a line for an electrolyte solution, in particular a calcium line (209).

15. A blood treatment apparatus (300), connected to at least a blood tubing set (200) according to any one of claims 11 to 14.

**Patentansprüche**

1. Einsatzstück (100) für ein Blutschlauchset (200), umfassend mindestens jeweils

   - eine erste Verbindungsstelle (101) zum Verbinden eines ersten Schlauchabschnitts (205a) des Blutschlauchsets (200) mit dem Einsatzstück (100);
   - eine zweite Verbindungsstelle (103) zum Verbinden eines zweiten Schlauchabschnitts (205b) des Blutschlauchsets (200) mit dem Einsatzstück (100);
   - eine dritte Verbindungsstelle (105) zum Verbinden eines dritten Schlauchabschnitts (209) des Blutschlauchsets (200) mit dem Einsatzstück (100);
   - eine erste Hauptleitung (107a) zum Leiten von Blut als eine erste Flüssigkeit durch das Einsatzstück (100), wobei die erste Hauptleitung (107a) in Fluidverbindung mit der ersten Verbindungsstelle (101) und mit der zweiten Verbindungsstelle (103) steht;
   - eine zweite Hauptleitung (107b) zum Leiten von Blut als die erste Flüssigkeit durch das Einsatzstück (100), wobei die zweite Hauptleitung (107b) in Fluidverbindung mit der ersten Verbindungsstelle (101) und mit der zweiten Verbindungsstelle (103) steht;
   - einen Verbindungsabschnitt (109), der die beiden Hauptleitungen (107a, 107b) miteinander oder mit der zweiten Verbindungsstelle (103) verbindet;
   - eine Sekundärleitung (111) zum Leiten einer zweiten Flüssigkeit, vorzugsweise einer Infusion, in mindestens eine/einen aus

      der ersten Hauptleitung (107a);
      die zweite Hauptleitung (107b); und
      dem Verbindungsabschnitt (109);
      wobei

   - die Sekundärleitung (111) in Fluidverbindung mit der dritten Verbindungsstelle (105) steht;
   - die erste Verbindungsstelle (101) ein durchströmbares Lumen mit einer ersten Querschnittsfläche aufweist;
   - die zweite Verbindungsstelle (103) ein durchströmbares Lumen mit einer zweiten Querschnittsfläche aufweist;
   - die erste Hauptleitung (107a) und/oder die zweite Hauptleitung (107b) ein durchströmbares Lumen mit einer dritten Querschnittsfläche aufweist; und
   - die erste Hauptleitung (107a) und die zweite Hauptleitung (107b) so angeordnet sind, dass Flüssigkeit durch das Einsatzstück (100) entweder durch die erste Hauptleitung (107a) oder die zweite Hauptleitung (107b) geleitet wird.

2. Das Einsatzstück (100) nach Anspruch 1, wobei die Summe einer Querschnittsfläche der ersten Hauptleitung (107a) und einer Querschnittsfläche der zweiten Hauptleitung (107b) kleiner ist als die Querschnittsfläche der ersten Verbindungsstelle (101), der zweiten Verbindungsstelle (103) und/oder der Querschnittsfläche des Schlauchabschnitts des mit dem Einsatzstück (100) verbundenen Blutschlauchsets (200).

3. Das Einsatzstück (100) nach Anspruch 1, wobei die Sekundärleitung (111) in Fluidverbindung mit einer Quelle für die Infusionslösung steht oder diese umfasst.

4. Das Einsatzstück (100) nach einem der vorhergehenden Ansprüche, wobei die dritte Querschnittsfläche maximal halb so groß oder im Wesentlichen halb so groß wie die erste Querschnittsfläche und/oder die zweite Querschnittsfläche ist.

5. Das Einsatzstück (100) nach einem der vorhergehenden Ansprüche, wobei der Verbindungsabschnitt (109) in Strömungsrichtung der ersten Flüssigkeit zwischen der ersten Hauptleitung (107a) und/oder der zweiten Hauptleitung (107b) einerseits und der zweiten Verbindungsstelle (103) und/oder dritten Verbindungsstelle (105) andererseits angeordnet ist.

6. Das Einsatzstück (100) nach einem der vorhergehenden Ansprüche, wobei der Verbindungsabschnitt (109) in Strömungsrichtung zur zweiten Verbindungsstelle (103) hin einen sich entlang der Strömungsrichtung erweiternden oder vergrößernden Querschnitt, insbesondere einen konischen, aufweist.

7. Das Einsatzstück (100) nach einem der vorhergehenden Ansprüche, wobei zwischen Abschnitten der ersten Hauptleitung (107a) und Abschnitten der zweiten Hauptleitung (107b) eine Durchgangsöffnung (130) des Einsatzstücks (100) angeordnet ist.

8. Das Einsatzstück (100) nach einem der vorhergehenden Ansprüche, bestehend aus zwei oder mindestens zwei Bauteilen, die mit- oder untereinander verschweißt und/oder verklebt sind.

9. Das Einsatzstück (100) nach einem der vorhergehenden Ansprüche, wobei die erste Hauptleitung (107a) und/oder die zweite Hauptleitung (107b) bzw. Abschnitte davon Schläuche sind, insbesondere solche, die in Schlaucheinführungen eingesetzt sind, die mit der ersten Verbindungsstelle (101) oder mit der zweiten Verbindungsstelle (103) in Fluidverbindung stehen und einstückig mit diesen hergestellt sind.

10. Das Einsatzstück (100) nach einem der vorhergehenden Ansprüche, wobei die erste Hauptleitung (107a) und/oder die zweite Hauptleitung (107b) integrale Bestandteile des Einsatzstücks (100) sind.

11. Blutschlauchset (200) mit mindestens einem Einsatzstück (100) nach einem der vorhergehenden Ansprüche, wobei das Einsatzstück (100) mit einer Zugabeleitung (207, 209) und mit einer Blutentnahmeleitung (203) und/oder mit einer Blutrückführleitung (205) in Fluidverbindung verbunden ist.

12. Das Blutschlauchset (200) nach Anspruch 11, das zur Durchführung einer regionalen Antikoagulation geeignet und/oder vorbereitet ist.

13. Das Blutschlauchset (200) nach einem der Ansprüche 11 bis 12, wobei das Blutschlauchset (200) zur Durchführung einer Hämodialyse, einer Hämofiltration, einer Hämodiafiltration, einer Plasmapheresebehandlung oder einer Vollblutadsorptionsbehandlung geeignet ist.

14. Das Blutschlauchset (200) nach einem der Ansprüche 11 bis 13, wobei die erste Verbindungsstelle (101) und die zweite Verbindungsstelle (103) mit einer Blutrückführleitung (205) des Blutschlauchsets (200) verbunden sind, und wobei die dritte Verbindungsstelle (105) mit einer Leitung für eine Elektrolytlösung, insbesondere einer Calciumleitung (209), verbunden ist.

15. Blutbehandlungsgerät (300), verbunden mit mindestens einem Blutschlauchset (200) nach einem der Ansprüche 11 bis 14.

## Revendications

1. Une pièce d'insertion (100) pour un set de tuyaux sanguins (200), comprenant au moins, respectivement:

- un premier site de raccordement (101) pour relier une première partie de tuyaux (205a) du set de tuyaux sanguins (200) à la pièce d'insertion (100);
- un second site de raccordement (103) pour relier une seconde partie de tuyaux (205b) du set de tuyaux sanguins (200) à la pièce d'insertion (100);
- un troisième site de raccordement (105) pour relier une troisième partie de tuyaux (209) du set de tuyaux sanguins (200) à la pièce d'insertion (100);
- un premier conduit principal (107a) pour conduire du sang en tant que premier liquide au travers de la pièce d'insertion (100), le premier conduit principal (107a) étant en communication fluidique avec le premier site de raccordement (101) ainsi qu'avec le second site de raccordement (103);
- un second conduit principal (107b) pour conduire du sang en tant que premier liquide au travers de la pièce d'insertion (100), le second conduit principal (107b) étant en communication fluidique avec le premier site de raccordement (101) ainsi qu'avec le second site de raccordement (103);
- une section de raccordement (109) reliant les deux conduits principaux (107a, 107b) l'un à l'autre ou au second site de raccordement (103);
- un conduit secondaire (111) pour conduire un second liquide, de préférence une infusion médicale, dans au moins l'un des éléments suivants:

le premier conduit principal (107a);
le second conduit principal (107b); et
la section de raccordement (109);
où

- le conduit secondaire (111) est en communication fluidique avec le troisième site de raccordement (105);
- le premier site de raccordement (101) comprend une lumière perfusable ayant une première zone de section transversale;
- le second site de raccordement (103) comprend une lumière perfusable ayant une seconde zone de section transversale;
- le premier conduit principal (107a) et/ou le second conduit principal (107b) comprend/comprennent une lumière perfusable ayant une troisième zone de section transversale; et

- le premier conduit principal (107a) ainsi que le second conduit principal (107b) sont agencés de telle sorte que du fluide soit conduit au travers de la pièce d'insertion (100) soit via le premier conduit principal (107a), soit via le second conduit principal (107b).

2. La pièce d'insertion (100) selon la première revendication, où la somme d'une zone de section transversale du premier conduit principal (107a) et d'une zone de section transversale du second conduit principal (107b) est inférieure à la zone de section transversale du premier site de raccordement (101), du second site de raccordement (103) et/ou de la zone de section transversale de la section de tuyaux du set de tuyaux sanguins (200) reliée à la pièce d'insertion (100).

3. La pièce d'insertion (100) selon la première revendication, où le conduit secondaire (111) est relié en communication fluidique à, ou comprend, une source pour la solution d'infusion médicale.

4. La pièce d'insertion (100) selon l'une quelconque des revendications précédentes, où la troisième zone de section transversale est au maximum moitié moins grande ou sensiblement moitié moins grande que la première zone de section transversale et/ou que la seconde zone de section transversale.

5. La pièce d'insertion (100) selon l'une quelconque des revendications précédentes, où la section de raccordement (109) est agencée dans le sens d'écoulement du premier liquide entre le premier conduit principal (107a) et/ou le second conduit principal (107b) d'une part, et le second site de raccordement (103) et/ou le troisième site de raccordement (105) d'autre part.

6. La pièce d'insertion (100) selon l'une quelconque des revendications précédentes, où la section de raccordement (109) comprend, dans le sens d'écoulement vers le second site de raccordement (103), une section transversale, notamment conique, qui s'élargit ou s'agrandit le long du sens d'écoulement.

7. La pièce d'insertion (100) selon l'une quelconque des revendications précédentes, où une ouverture traversante (130) de la pièce d'insertion (100) est agencée entre les sections du premier conduit principal (107a) et les sections du second conduit principal (107b).

8. La pièce d'insertion (100) selon l'une quelconque des revendications précédentes, constituée de deux ou d'au moins deux composants soudés et/ou collés les uns aux autres.

9. La pièce d'insertion (100) selon l'une quelconque des revendications précédentes, où le premier conduit principal (107a) et/ou le second conduit principal (107b), ou des sections de ceux-ci, respectivement, sont des tuyaux, notamment ceux qui sont insérés dans des entrées de tuyaux en communication fluidique avec le premier site de raccordement (101) ou avec le second site de raccordement (103) et sont fabriqués intégralement avec celui-ci.

10. La pièce d'insertion (100) selon l'une quelconque des revendications précédentes, où le premier conduit principal (107a) et/ou le second conduit principal (107b) font partie intégrante de la pièce d'insertion (100).

11. Un set de tuyaux sanguins (200) comprenant au moins une pièce d'insertion (100) selon l'une quelconque des revendications précédentes, la pièce d'insertion (100) étant reliée en communication fluidique à un conduit d'ajout (207, 209) ainsi qu'à un conduit de prélèvement sanguin et/ou à un conduit de prélèvement sanguin (203) et/ou à un conduit de retour sanguin (205).

12. Le set de tuyaux sanguins (200) selon la revendication 11 adapté et/ou préparé pour l'exécution d'une anticoagulation régionale.

13. Le set de tuyaux sanguins (200) selon l'une quelconque des revendications 11 à 12, où le set de tuyaux sanguins (200) est adapté à l'exécution d'une hémodialyse, d'une hémofiltration, d'une hémodiafiltration, d'un traitement plasmaphérèse ou d'un traitement d'adsorption de la totalité du sang.

14. Le set de tuyaux sanguins (200) selon l'une quelconque des revendications 11 à 13, où le premier site de raccordement (101) et le second site de raccordement (103) sont reliés à un conduit de retour sanguin (205) du set de tuyaux sanguins (200), et où le troisième site de raccordement (105) est relié à un conduit pour une solution d'électrolyte, notamment à un conduit de calcium (209).

15. Un appareil de traitement du sang (300) relié à au moins un set de tuyaux sanguins (200) selon l'une quelconque des revendications 11 à 14.

Fig. 1

Fig. 2

Fig. 3

Fig. 4

Fig. 5

Fig. 6

101

101a

107b

130

103a

103

107a

115

105

Fig. 7

Fig. 8

Fig. 9

Fig. 10

Fig. 11

Fig. 12

105

103a

107b        107a

## Fig. 13a

105

101a

103

107a        107b

## Fig. 13b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014026771 A1
- US 20100168643 A1
- US 7931612 B2
- US 20130150772 A1
- WO 2018055091 A1
- DE 102013011010 A1